(19) Europäisches Patentamt
European Patent Office
Office européen des brevets

(11) Numéro de publication : **0 332 551 B1**

(12) # FASCICULE DE BREVET EUROPEEN

(45) Date de publication du fascicule du brevet : **21.04.93 Bulletin 93/16**

(51) Int. Cl.$^5$ : **A61K 7/20,** A61K 7/30, A61L 2/00, A01N 59/00

(21) Numéro de dépôt : **89440011.8**

(22) Date de dépôt : **10.02.89**

(54) **Formes galéniques solides contenant au moins une substance peroxydique ainsi qu'un sel de métal de transition.**

(30) Priorité : **11.02.88 FR 8801781**

(43) Date de publication de la demande : **13.09.89 Bulletin 89/37**

(45) Mention de la délivrance du brevet : **21.04.93 Bulletin 93/16**

(84) Etats contractants désignés : **AT BE CH DE ES FR GB GR IT LI LU NL SE**

(56) Documents cités :
**EP-A- 0 255 041**
**WO-A-87/01562**
**FR-A- 383 839**
**FR-A- 2 325 363**
**FR-A- 2 400 906**
**GB-A- 2 047 091**
**US-A- 2 498 343**
**CHEMICAL ABSTRACTS, vol. 83, 1975, page 361, résumé no. 84731p, Columbus, Ohio, US; & SE-A-367 319 (ASTRA LAKEMEDEL AB) 27-05-1974**
**IDEM**
**CHEMICAL ABSTRACTS, vol. 108, 1988, page 128, résumé no. 40133a, Columbus, Ohio, US; & JP-A-62 195 100 (LION CORP.) 27-08-1987**

(73) Titulaire : **RECHERCHE- CONCEPTION- DEVELOPPEMENT "R.C.D. LABO" S.à.r.l.**
**Z.I. Krafft**
**F-67150 Erstein (FR)**

(72) Inventeur : **Jung,Christophe**
**205 route d'Oberhausbergen**
**67200 Strasbourg (FR)**
Inventeur : **Jung,Jean**
**205, route d'Oberhausbergen**
**67200 Strasbourg (FR)**
Inventeur : **Touitou,Norbert**
**13,Boulevard Tauler**
**67000 Strasbourg (FR)**
Inventeur : **Ollinger, Bernard**
**4, rue Lucius**
**67100 Strasbourg (FR)**
Inventeur : **Banzet Philippe**
**15, rue Pertois**
**67100 Strasbourg (FR)**
Inventeur : **Jung,Simone**
**205, route d'Oberhausbergen**
**67200 Strasbourg (FR)**
Inventeur : **Toulouse,Jean-Jacques**
**1,rue de Boston**
**67000 Strasbourg (FR)**

(74) Mandataire : **Nuss, Pierre et al**
**10, rue Jacques Kablé**
**F-67080 Strasbourg Cédex (FR)**

EP 0 332 551 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des formes galéniques solides contenant au moins une substance peroxydique ainsi qu'un sel de métal de transition. Elle a également pour objet un procédé pour l'obtention de telles formes galéniques solides ainsi que différentes utilisations desdites formes galéniques solides.

On sait que les résidus d'aliments forment progressivement sur les dents, et au contact de la salive, un film invisible : la plaque dentaire, milieu propice au développement bactérien responsable de la carie. De plus, après la consommation d' épices, d'alcool, de fumée de cigarettes avec leurs goudrons, il se dégage une mauvaise haleine désagréablement ressentie, due à la formation de substances fétides dans la sphère buccale. Les fumeurs observent un jaunissement prématuré des dents, provoqué par la fumée et les composés de la cigarette portés à très haute température, conduisant à la formation de goudrons.

Il existe des thiocyanates ($SCN^-$) présents physiologiquement dans la salive. En outre, les thiocyanates sont oxydés sous l'influence de peroxydase, la lactoperoxydase, avec formation d'hypothiocyanate. La plaque dentaire est inhibée par la peroxydase salivaire présentant un système antibactérien. Le système antibactérien salivaire reposant sur la lactoperoxidase, les thiocyanates et le peroxyde d'hydrogène sont actuellement utilisés dans la prévention des caries sous forme de pâtes et solutions dentifrices.

Les peroxydes physiologiques sont en assez faibles concentrations dans la salive, ce qui limite par conséquent la formation des hypothiocyanates, à l'origine des propriétés antibactériennes, anti-plaque dentaire, inhibitrices du métabolisme des hydrates de carbone en acides responsables de la plaque dentaire, blanchissante. D'autre part, l'emploi de pâtes et solutions dentifrices n'est pas commode et empêche une utilisation courante en dehors de sa salle de bains.

On connaît du document Chemical Abstracts vol. 83, 1975, page 361, un dentifrice moussant contenant trois éléments, à savoir de l'acide ascorbique (milieu réducteur), du percarbonate de sodium (oxydant puissant) et du sulfate de cuivre (catalyseur métallique). Mais ce dentifrice moussant ne permet nullement d'obtenir le résultat souhaité, notamment de purifier et de rafraîchir la mauvaise haleine et de blanchir les dents.

On connaît, par ailleurs, de FR-A-2 400 906 un système de nettoyage de lentilles comprenant un composé ène-diol et un oxydant, c'est-à-dire un système rédox composé d'un oxydant puissant et, par exemple, d'un acide ascorbique, ainsi qu'éventuellement d'un catalyseur métallique. Mais ce système de nettoyage de lentilles ne permet pas la dégradation des micro-organismes des lentilles ophtalmiques.

On connaît également du document Chemical Abstracts vol. 108, 1988, page 128, un système formé d'un agent blanchissant, c'est-à-dire un oxydant puissant, d'acides organiques, ainsi que des sels catalytiques. Or les acides organiques présents neutralisent l'activité de l'oxydant et entraînent ainsi principalement la formation de gaz carbonique, c'est-à-dire empêchant l'oxydation des thiocyanates.

Enfin, on connaît du document US-A-2 498 343 un procédé de nettoyage de dents artificielles, ainsi que de dentiers. Ce document repose, par conséquent, sur un système nettoyant in vitro et non in vivo. Le système est composé d'un oxydant et d'un agent activateur. Or, aucun ajout de thiocyanates n'est prévu. Ce système ne permet donc pas non plus la formation d'hypothiocyanates.

La présente invention a pour but de pallier ces inconvénients.

Elle a, en effet, pour objet des formes galéniques solides caractérisées en ce qu'elles contiennent au moins une substance peroxydique libérant de l'oxygène natif et actif, sous forme de perhydrol, agissant sur des thiocyanates physiologiquement présents en milieu salivaire, ou préalablement rajoutés en milieu soit aqueux, soit en solution dont le pH est préférentiellement alcalin pour les percarbonates alcalins, de manière à former des hypothiocyanates, ainsi qu'un sel de métal de transition sous la forme d'un sel de fer conduisant à la formation, d'une part, de différents radicaux libres, en particulier, des radicaux hydroxyles $OH\cdot$, oxydants très puissants, et, d'autre part, d'ions ferry et perferryl dégradant les phospholipides, les acides aminés, les protéines, les hydrates de carbone et éliminant les germes et les micro-organismes tels les champignons, diminuant ainsi le développement bactérien, le métabolisme des sucres, dont le saccharose, et la formation des composés acides, lesdites formes galéniques ne contenant pas de composés ène-diols, ces formes galéniques étant utilisables en hygiène bucco-dentaire, ou applicables, comme agent de nettoyage et/ou de décontamination, à l'entretien de tout élément destiné à être mis en contact avec des muqueuses de l'homme ou de l'animal, notamment pour l'entretien de lentilles ophtalmiques.

L'invention sera mieux comprise grâce à la description ci-après, qui se rapporte à un mode de réalisation préféré.

Selon une caractéristique de l'invention, l'oxygène natif et actif existe sous différentes formes réactives, telles l'anion superoxyde $O_2^{\cdot -}$, le radical perhydroxyle $HO_2\cdot$, le peroxyde d'hydrogène $H_2O_2$, le radical hydroxyle $HO\cdot$, l'oxygène singulet $^1O_2$, le radical organique peroxy $ROO^\cdot$, l'hydroperoxyde organique ROOH, le radical organique alcoxyle $RO^\cdot$, le carbonyle excité RO*.

Selon une première variante de réalisation de l'invention, chaque substance peroxydique (agent oxygénateur) est sous la forme soit d'un acide peroxycarbonique, d'un acide peroxysulfurique, d'un acide peroxyphosphorique, d'un acide perborique et leurs dérivés de salification et/ou d'addition, soit de peroxydes tels le peroxyde de calcium, le peroxyde de magnésium, le peroxyde de fer, le peroxyde de manganèse, le peroxyde de sodium, ou encore le peroxyde de zinc.

Selon une seconde variante de réalisation de l'invention, chaque substance peroxydique (agent oxygénateur) est sous la forme de composés d'addition entre carbonates ordinaires et l'eau oxygénée tels le percarbonate de potassium ou de sodium $2Na_2CO_3, 3H_2O_2$ encore appelés perhydrates de carbonates de potassium ou de sodium.

Conformément à l'invention, l'une des formes réactives de l'oxygène natif et actif, sous la forme de perhydrol, obtenue à partir d'au moins une substance peroxydique, agit sur des thiocyanates, de manière à former des hypothiocyanates, selon la réaction suivante:

$$H_2O_2 + SCN^- \xrightarrow[\text{lactoperoxydase}]{} OSCN^- + H_2O$$

Si l'oxygène natif et actif est libéré en milieu salivaire, les hypothiocyanates ($OSCN^-$) sont formés uniquement par les thiocyanates physiologiquement présents dans la salive. La réaction sera alors catalysée par une enzyme présente dans la salive, la lactoperoxydase. Si, par contre, il est libéré en milieu aqueux ou en un milieu dont le pH est préférentiellement alcalin pour les percarbonates alcalins, les hypothiocyanates ($OSCN^-$) sont uniquement formés par réaction sur des thiocyanates préalablement rajoutés.

L'hypothiocyanate ainsi formé étant un inhibiteur bactérien, il freine le développement bactérien et inhibe la production d'acides responsables de la formation de la plaque dentaire. De même, il restreint le métabolisme du sucre et la formation d'acides par fermentation. De plus, le peroxyde d'hydrogène ($H_2O_2$) se décompose dans la cavité buccale et au contact de la salive pour donner de l'oxygène ($O_2$ et ses métabolites) selon la réaction suivante :

$$H_2O_2 \rightarrow H_2O + 1/2\ O_2$$

L'oxygène ainsi dégagé empêche également la prolifération bactérienne et freine le développement des bactéries anaérobies responsables des odeurs fétides impliquées dans la mauvaise haleine. L'oxygène natif ainsi présent au sein de la cavité buccale, purifie et rafraîchit la mauvaise haleine, et blanchit les dents.

Conformément à l'invention, les formes galéniques solides pourront être avantageusement sous la forme soit de comprimés, soit de tablettes, soit de pastilles, soit de gélules, soit encore de poudres simples ou composées.

La présente invention a également pour objet un procédé pour l'obtention desdites formes galéniques solides, caractérisé en ce qu'il consiste en une compression, à température et humidité contrôlées, d'un mélange d'un granulé, éventuellement préparé par voie humide, d'arômes, de silice colloïdale hydrophobe et hydrophile servant d'excipient, de substances peroxydiques et enfin d'un sel de métal de transition sous forme d'un sel de fer, lesdites formes galéniques ne contenant pas de composés ène-diols.

Préférentiellement, on utilise avantageusement comme substance peroxydique, du percarbonate de sodium à raison de 10 %, et comme sel de métal de transition, du sel de fer ferreux à raison de 3 %.

Ce sel de métal de transition pourra être avantageusement sous la forme de sulfate ferreux.

Afin de mieux comprendre le déroulement du procédé de fabrication des formes galéniques solides conformes à l'invention, par compression directe d'un mélange ou par granulation humide de l'excipient, on donne ci-après deux exemples illustrant la séquence d'un tel procédé :

<u>Exemple 1</u> : (par compression directe)

Pour un lot de 100 kg : dissoudre 0,2 kg de menthol dans 1 kg d'huile essentielle, de menthe par exemple, par agitation mécanique. Mélanger avec 18 kg de saccharose broyé finement. Ajouter comme excipient 0,7 kg de silice colloïdale hydrophile. Homogénéiser et ajouter comme excipient 0,3 kg de silice colloïdale hydrophobe.

Homogénéiser. Dans un grand fût en acier inoxydable, ajouter 62,8 kg de dextrose, 2 kg d'acide citrique anhydre, 10 kg de percarbonate de sodium et 3 kg de sulfate de fer ferreux. Bien mélanger pendant trente minutes. Ajouter l'arôme dont la préparation est décrite ci-dessus, puis bien mélanger pendant dix minutes. Ajouter 2 kg de stéarate de magnésium. Bien mélanger pendant deux minutes.

Le saccharose et le dextrose peuvent être remplacés par du lactose et de l'aspartam. Comprimer par exemple sur une machine à comprimer rotative en atmosphère contrôlée (environ trente pour cent d'humidité relative

à une température de 20°C). Conditionner immédiatement, en atmosphère contrôlée (30 % d'humidité relative pour 20°C) dans des complexes blisters étanches ou des flacons en plastique étanches, PVDC ou PVC par exemple, ou de verre par exemple, munis d'une capsule déshydratante.

Exemple 2 : (par granulation humide)

Pour un lot de 100 kg : dissoudre 0,2 kg de menthol dans 1 kg d'huile essentielle, de menthe par exemple, par agitation mécanique. Ajouter 14,51 kg de saccharose finement broyé. Bien mélanger. Ajouter comme excipient 0,7 kg de silice colloïdale hydrophile. Bien mélanger et ajouter comme excipient 0,3 kg de silice colloïdale hydrophobe. Bien mélanger. Réaliser un sirop en dissolvant, par agitation mécanique, 3,49 kg de saccharose et 2 kg d'acide citrique dans 3,03 kg d'eau purifiée. Dans un fût étanche, ajouter 62,8 kg de dextrose, ajouter le sirop ; bien mélanger pendant trente minutes. Granuler sur un granulateur, oscillant, par exemple, en atmosphère de température et d'humidité contrôlées (température 20°C, humidité relative 30 %). Recueillir le granulé sur des clayettes en couches fines. Strier le granulé pour permettre l'évaporation de l'humidité. Sécher à l'étuve à 40°C - 50°C pendant douze heures. Granuler le granulé sec au granulateur oscillant, par exemple, en atmosphère contrôlée (20°C et 30 % humidité relative). Toujours en atmosphère contrôlée, ajouter dans un fût étanche le granulé, l'arôme, 3 kg de sulfate de fer ferreux et 10 kg de percarbonate de sodium. Bien mélanger pendant trente minutes. Ajouter 2 kg de stéarate de magnésium. Bien mélanger pendant trois minutes. Le saccharose et le dextrose peuvent être remplacés par du lactose et de l'aspartam.

Comprimer sur une machine à comprimer rotative, en atmosphère contrôlée (20°C, 30 % d'humidité relative). Conditionner immédiatement, en atmosphère contrôlée (30 % humidité relative pour 20°C) dans des complexes blisters étanches ou des flacons en plastique étanches, PVDC par exemple, ou de verre par exemple, munis d'une capsule déshydratante.

## Revendications

1. Formes galéniques solides, caractérisées en ce qu'elles contiennent au moins une substance peroxydique libérant de l'oxygène natif et actif, sous forme de perhydrol, agissant sur des thiocyanates physiologiquement présents en milieu salivaire, ou préalablement rajoutés en milieu soit aqueux, soit en solution dont le pH est préférentiellement alcalin pour les percarbonates alcalins, de manière à former des hypothiocyanates, ainsi qu'un sel de métal de transition sous la forme d'un sel de fer conduisant à la formation, d'une part, de différents radicaux libres, en particulier, des radicaux hydroxyles $OH\cdot$, oxydants très puissants, et, d'autre part, d'ions ferry et perferryl dégradant les phospholipides, les acides aminés, les protéines, les hydrates de carbone et éliminant les germes et les micro-organismes tels les champignons, diminuant ainsi le développement bactérien, le métabolisme des sucres, dont le saccharose, et la formation des composés acides, lesdites formes galéniques ne contenant pas de composés ène-diols, ces formes galéniques étant utilisables en hygiène bucco-dentaire, ou applicables, comme agent de nettoyage et/ou de décontamination, à l'entretien de tout élément destiné à être mis en contact avec des muqueuses de l'homme ou de l'animal.

2. Formes galéniques solides selon la revendication 1, caractérisées en ce que l'oxygène natif et actif existe sous différentes formes réactives, telles l'anion superoxyde $O_2^{\cdot -}$, le radical perhydroxyle $HO_2\cdot$, le peroxyde d'hydrogène $H_2O_2$, le radical hydroxyle $HO\cdot$, l'oxygène singulet $^1O_2$, le radical organique peroxy $ROO\cdot$, l'hydroperoxyde organique ROOH, le radical organique alcoxyle $RO\cdot$, le carbonyle excité RO*.

3. Formes galéniques solides selon l'une quelconque des revendications 1 et 2, caractérisées en ce que chaque substance peroxydique est sous la forme soit d'un acide peroxycarbonique, d'un acide peroxysulfurique, d'un acide peroxyphosphorique, d'un acide perborique et leurs dérivés de salification et/ou d'addition, soit de peroxydes tels le peroxyde de calcium, le peroxyde de magnésium, le peroxyde de fer, le peroxyde de manganèse, le peroxyde de sodium, ou encore le peroxyde de zinc.

4. Formes galéniques solides selon l'une quelconque des revendications 1 et 2, caractérisées en ce que chaque substance peroxydique est sous la forme de composés d'addition entre carbonates ordinaires et l'eau oxygénée tels le percarbonate de potassium ou de sodium $2Na_2CO_3$, $3H_2O_2$ encore appelés perhydrates de carbonates de potassium ou de sodium.

5. Formes galéniques solides selon la revendication 1, caractérisées en ce que les hypothiocyanates sont

formés, en milieu salivaire, uniquement par réaction sur les thiocyanates physiologiquement présents dans la salive en présence de l'enzyme lactoperoxidasique.

6. Formes galéniques solides selon la revendication 1, caractérisées en ce que les hypothiocyanates sont formés en milieu soit aqueux, soit en solution dont le pH est préférentiellement alcalin pour les percarbonates alcalins, uniquement par réaction sur des thiocyanates préalablement rajoutés.

7. Formes galéniques solides selon l'une quelconque des revendications 1 à 6, caractérisées en ce qu'elles sont avantageusement sous la forme soit de comprimés, soit de tablettes, soit de pastilles, soit de gélules, soit encore de poudres simples ou composées.

8. Procédé pour l'obtention de formes galéniques solides selon l'une quelconque des revendications 1 à 7, caractérisé en ce qu'il consiste en une compression, à température et humidité contrôlées, d'un mélange d'un granulé, éventuellement préparé par voie humide, d'arômes, de silice colloïdale hydrophobe et hydrophile servant d'excipient, de substances peroxydiques et enfin d'un sel de métal de transition sous la forme d'un sel de fer, lesdites formes galéniques ne contenant pas de composés ène-diols.

9. Procédé pour l'obtention de formes galéniques solides selon la revendication 8, caractérisé en ce qu'on utilise avantageusement comme substance peroxydique, du percarbonate de sodium à raison de 10 %, et comme sel de métal de transition, du sel de fer ferreux à raison de 3 %.

10. Procédé pour l'obtention de formes galéniques solides selon la revendication 9, caractérisé en ce que le sel de métal de transition est avantageusement sous la forme de sulfate ferreux.

11. Utilisation de formes galéniques solides selon l'une quelconque des revendications 1 à 7, obtenues par application du procédé selon l'une quelconque des revendications 9 à 10, comme agent de nettoyage et/ou de décontamination, pour l'entretien de lentilles ophtalmiques.

## Patentansprüche

1. Feste galenische Zubereitungsformen, **dadurch gekennzeichnet,** daß sie mindestens eine peroxidische Substanz enthalten, die nativen und aktiven Sauerstoff unter Bildung von Perhydrol freisetzt, das gegenüber physiologischen Thiocyanaten wirksam ist, die im Speichelmillieu vorhanden sind oder vorher hinzugefügt wurden, entweder in wäßrigem Milieu oder in Lösung mit einem vorzugsweise alkalischen pH-Wert aufgrund darin enthaltender Alkalipercarbonate derart, daß Hypothiocyanate gebildet werden, sowie auch ein Salz eines Übergangsmetalls enthalten, in Form eines Eisensalzes, das einerseits die Bildung verschiedener freier Radikale, insbesondere von Hydroxylradikalen OH· als sehr kräftige Oxidationsmittel, und andererseits von Ferriionen und Perferrylionen steuert, die die Phospholipide, die Aminosäuren, die Proteine und die Kohlenhydrate abbauen und die Keime und die Mikroorganismen, wie die Pilze, eliminieren und ebenfalls die Entwicklung von Bakterien und dem Zuckerstoffwechsel, darunter den von Saccharose und die Bildung saurer Stoffe vermindert, wobei diese galenischen Zubereitungsformen keine En-diole enthalten und diese galenischen Zubereitungsformen bei der Mundhygiene und Zahnpflege brauchbar sind oder als Reinigungsmittel und/oder Entgiftungsmittel zur Instandhaltung aller Elemente, die zum Kontakt mit Schleimhäuten des Menschen oder Tieres bestimmt sind, anwendbar sind.

2. Feste galenische Zubereitungsformen nach Anspruch 1, **dadurch gekennzeichnet,** daß der native und aktive Sauerstoff in verschiedenen reaktiven Formen vorliegt, wie im Superoxid-Anion $O_2^{-}$, im Perhydroxylradikal $HO_2$·, dem Wasserstoffperoxid $H_2O_2$, dem Hydroxylradikal HO·, dem Singulett-Sauerstoff $^1O_2$, dem organischen Peroxgradikal ROO·, dem organischen Hydroperoxid ROOH, dem organischen Alkoxylradikal RO·, dem angeregten Carbon 1 RO*.

3. Feste galenische Zubereitungsformen nach einem der Ansprüche 1 und 2, **dadurch gekennzeichnet,** das jede peroxidische Substanz entweder in Form einer Peroxycarbonsäure, einer Peroxyschwefelsäure, einer Peroxyphosphorsäure, einer Peroxyborsäure und deren Derivate der Salzbildung und/oder Addition, oder als Peroxide, wie Calciumperoxid, Magnesiumperoxid, Eisenperoxid, Manganperoxid, Natriumperoxid oder Zinkperoxid vorliegt.

4. Feste galenische Zubereitungsformen nach einem der Ansprüche 1 und 2, **dadurch gekennzeichnet,**

daß jede peroxidische Substanz in form einer Additionsverbindung zwischen gewöhnlichen Carbonaten und Wasserstoffperoxidlösung, wie Kalium- oder Natriumpercarbonat 2 $Na_2CO_3$, 3 $H_2O_2$, auch als Carbonat-perhydrate von Kalium oder Natrium bezeichnet, vorliegt.

5. Feste galenische Zubereitungsformen nach Anspruch 1, **dadurch gekennzeichnet,** daß die Hypothiocyanate im Speichelmilieu allein durch Reaktion der physiologischen Thiocyanate, die im Speichel vorhanden sind, in Gegenwart des Ferments Lactoperoxidase gebildet worden sind.

6. Feste galenische Zubereitungsformen nach Anspruch 1, **dadurch gekennzeichnet,** daß die Hypothiocyanate entweder im wäßrigen Milieu oder in einer Lösung, deren pH-Wert vorzugsweise alkalisch aufgrund von Alkalipercarbonaten ist, allein durch Reaktion mit vorzugsweise hinzugefügten Thiocyanaten gebildet worden sind.

7. Feste galenische Zubereitungsformen nach einem der Ansprüche 1 bis 6, **dadurch gekennzeichnet,** daß sie bevorzugt entweder in gepreßter Form, wie als Tabletten, oder in Form von Pastillen oder als Gelulen, oder als einfache oder zusammengesetzte Pulver vorliegen.

8. Verfahren zur Herstellung von festen galenischen Zubereitungsformen nach einem der Ansprüche 1 bis 7, **dadurch gekennzeichnet,** daß es ein Verpressen bei kontrollierter Temperatur und Feuchtigkeit einer Mischung eines Granulats, das ggf. auf nassem Wege hergestellt wurde, von Aromen, von kolloidaler hydrophober und hydrophiler Kieselsäure, die als Exzipient dient, von peroxidischen Substanzen und schließlich einem Übergangsmetallsalz in Form eines Eisensalzes umfaßt, wobei diese galenischen Zubereitungsformen keine En-diolverbindungen enthalten.

9. Verfahren zur Herstellung von festen galenischen Zubereitungsformen nach Anspruch 8, **dadurch gekennzeichnet,** daß man vorzugsweise als peroxidische Substanz Natriumpercarbonat mit etwa 10 % und als Übergangsmetallsalz das Ferroeisensalz mit etwa 3 % verwendet.

10. Verfahren zur Herstellung von festen galenischen Zubereitungsformen nach Anspruch 9, **dadurch gekennzeichnet,** daß das Übergangsmetallsalz vorzugsweise in der Form von Ferrosulfat vorliegt.

11. Verfahren zur Herstellung von festen galenischen Zubereitungsformen nach einem der Ansprüche 1 bis 7, erhalten durch Anwendung des Verfahrens nach einem der Ansprüche 9 bis 10, als Mittel zur Reinigung und/oder Entgiftung, für die Instandhaltung von Augen-Kontaktlinsen.

## Claims

1. Solid galenical forms, characterised in that they contain at least one peroxidic substance liberating native, active oxygen in the form of perhydrol, acting on the thiocyanates physiologically present in the salivary medium, or previously added either in an aqueous medium or in a solution of which the pH is preferably alkaline in the case of alkaline percarbonates so as to form hypothiocyanates, as well as a transition metal salt in the form of an iron salt leading to the formation, on the one hand, of various free radicals, in particular hydroxyl radicals OH· which are very powerful oxidising agents and, on the other hand, of ferry and perferryl ions which degrade phospholipids, amino-acids, proteins and carbohydrates and eliminate the bacteria and micro-organisms such as fungi , thus reducing bacterial growth, the metabolism of sugars, including saccharose, and the formation of acidic compounds, said galenical forms not containing enediol compounds, these galenical forms being usable in buccal-dental hygiene or being applicable as a cleaning and/or decontaminating agent to the maintenance of any element intended to come into contact with human or animal mucous membranes.

2. Solid galenical forms according to claim 1, characterised in that native, active oxygen exists in different reactive forms such as the superoxide anion $O_2^{\cdot -}$ , the perhydroxyl radical $HO_2\cdot$, hydrogen peroxide $H_2O_2$, the hydroxy radical HO·, singlet oxygen $^1O_2$, the peroxyorganic radical ROO·, the organic hydroperoxide ROOH, the organic alkoxy radical RO·, the excited carbonyl RO*.

3. Solid galenical forms according to any one of claims 1 and 2, characterised in that each peroxide substance is in the form of a peroxycarbonic acid, a peroxysulphuric acid, a peroxyphosphoric acid, a perboric acid and salification and/or addition derivatives thereof, or peroxides such as calcium peroxide, magnesium peroxide, iron peroxide, manganese peroxide, sodium peroxide or again zinc peroxide.

4. Solid galenical forms according to any one of claims 1 and 2, characterised in that each peroxidicsubstance is in the form of compounds of addition between ordinary carbonates and hydrogen peroxide such as potassium or sodium percarbonate $2Na_2CO_3$, $3H_2O_2$ also known as potassium or sodium carbonate perhydrates.

5. Solid galenical forms according to claim 1, characterised in that the hypothiocyanates are formed, in a salivary medium, solely by reaction with the thiocyanates physiologically present in the saliva in the presence of the lactoperoxidasic enzyme.

6. Solid galenical forms according to claim 1, characterised in that the hypothiocyanates are formed either in an aqueous medium or in a solution of which the pH is preferably alkaline in the case of alkaline percarbonates, solely by reaction with previously added thiocyanates.

7. Solid galenical forms according to any one of claims 1 to 6, characterised in that they are advantageously in the form of compacts, tablets, pastilles, capsules, or simple or compound powders.

8. Process for obtaining solid galenical forms according to any one of claims 1 to 7, characterised in that it involves compression at controlled temperature and humidity of a mixture of a granulate, optionally prepared by humid means, flavourings, hydrophobic and hydrophilic colloidal silica acting as excipient, peroxidic substances and finally a transition metal salt in the form of an iron salt, said galenical forms not containing enediol compounds.

9. Process for obtaining solid galenical forms according to claim 8, characterised in that it is advantageous to use 10% sodium percarbonate as peroxidic substance and 3% ferrous salt as transition metal salt.

10. Process for obtaining solid galenical forms according to claim 9, characterised in that the transition metal salt is advantageously in the form of ferrous sulphate.

11. Use of solid galenical forms according to any one of claims 1 to 7 obtained by application of the process according to any one of claims 9 to 10 as cleaning and/or decontaminating agent for the maintenance of opthalmic lenses.